# EUROPÄISCHE PATENTSCHRIFT

(11) **EP 0 447 744 B1**
(45) Veröffentlichungstag und Bekanntmachung des Hinweises auf die Patenterteilung: **12.03.1997**
(21) Anmeldenummer: 91100259.0
(22) Anmeldetag: 10.01.1991
(51) Int. Cl.: A61F 2/30, A61L 27/00

(54) **Verfahren zur Herstellung von osteointegrierenden Oberflächen auf Skelettimplantaten sowie Skelettimplantat**
Method for producing osteointegrating surfaces of sceletal implants as well as sceletal implant
Procédé pour fabriquer des surfaces ostéointégrantes d'implants du squelette aussi bien qu'un implant du squelette

(30) Priorität: 23.03.1990 DE 4009337
(43) Veröffentlichungstag der Anmeldung: 25.09.1991
(73) Patentinhaber: Repenning, Detlev, Dr., D-21465 Reinbek (DE)
(72) Erfinder: Repenning, Detlev, Dr., D-21465 Reinbek (DE)
(74) Vertreter: Niedmers, Ole, Dipl.-Phys.

(56) Entgegenhaltungen:
- EP-A- 0 211 676
- EP-A- 0 264 353
- WO-A-86/06617
- FR-A- 2 383 656
- US-A- 4 483 678

## Beschreibung

Die Erfindung betrifft ein Verfahren zur Herstellung von osteointegrierenden Oberflächen auf Skelettimplantaten aus Metall oder Metallegierungen, wobei zuerst eine Oxidschicht auf dem Skelettimplantat aufgebracht wird und nachfolgend darauf eine Calciumhydroxylapatitschicht, sowie ein Skelettimplantat.

Ein Verfahren sowie ein Skelettimplantat dieser Art sind bekannt (EP-A- 0 211 676). Nach diesem bekannten Verfahren wird auf einem Substrat aus Titan eine Oxidschicht erzeugt, in dem beispielsweise das metallische Substrat mittels 400 bis 800 °C warmer Luft erwärmt wird und zwar über ein Zeitintervall von 1 bis 10 Minuten. Nachfolgend wird bei diesem Verfahren die nächste Schicht aus keramischem Werkstoffen mittels eines thermischen Plasmaspritzvorganges ausgebildet. Diese Schicht aus keramischen Werkstoff kann Hydroxylapatit enthalten. Aufgrund dieser Verfahrensführung kann sich nur eine unzureichende dicke Oxidschicht ausbilden, deren mechanische Widerstandsfähigkeit für die hiesigen Zwecke als nicht ausreichend groß anzusehen ist.

Neben einer hohen Festigkeit kommt es bei Skelettimplantaten wesentlich darauf an, daß sie mit dem sie umgebenden biologischen Milieu verträglich sind. Es sind grundsätzlich seit langem Implantatwerkstoffe aus Metall oder Metallegierungen bekannt, die eine Oxidschicht aufweisen, die sich beispielsweise beim Einsatz des Implantats in das biologische Milieu in diesem mehr oder weniger zufällig selbst ausbildet. So sind beispielsweise bisher Skelettimplantate aus Titan unbeschichtet implantiert worden, wobei durch Reaktion im biologischen Milieu sich auf dem Titanwerkstoff eine Titandioxydschicht mehr oder weniger zufällig ausgebildet hat. Man hat aber auch versucht, durch anodischen Auftrag Titandioxydschichten auf den Titanimplantaten vor der Implantierung auszubilden, wobei Titandioxydschichten mit einer Schichtdicke von ca. 4 x 10⁻⁷ bis 7 x 10⁻⁷ m aufgebracht wurden.

Es hat sich jedoch gezeigt, daß diese sich zufällig aufbauenden Schichten nur unzureichend dünn, porenreich und beschädigungsanfällig sind. Dieses gilt gleichermaßen für die anodisierten Titanoxydschichten. Ist eine Oxidschicht erst einmal verletzt, führt dieses zur Reibkorrosion und Bildungen von Titanverbindungen mit niedriger Oxidationsstufe im Körper, die in der Regel nekrotisch wirken.

Ein weiteres Problem bei den bekannten Skelettimplantaten besteht darin, daß sich das Skelettimplantat vielfach nicht auf gewünschte Weise mit dem Knochen, in den das Skelettimplantat eingesetzt wird, verbindet. Man hat zwar versucht, dieses Problem dadurch zu lösen, daß man das Implantat selbst in bezug auf seine Oberfläche porös ausbildete, um die Haft- bzw. Anwachsoberfläche des Implantats zu vergrößern, diese Maßnahme führte jedoch nicht in allen Fällen zu einem Erfolg und erwies sich in vielen Fällen auch nicht in bezug auf die gewünschte Verbindungsfestigkeit mit dem Knochen als ausreichend.

Es ist Aufgabe der vorliegenden Erfindung ein Verfahren zu schaffen, mit dem Oxidschichten auf Skelettimplantaten aufgebracht werden können, die einerseits besonders gute Integrationseigenschaften im biologischen Milieu aufweisen, die gleichmäßig aufbaubar und mechanisch widerstandsfähig sein sollen, und die andererseits sehr gute osteointegrierende Eigenschaften aufweisen, wobei das Verfahren verhältnismäßig leicht ausführbar sein soll, und daß ein Skelettimplantat geschaffen werden soll, das die vorstehend aufgeführten Eigenschaften aufweist.

Gelöst wird die Aufgabe gemäß dem erfindungsgemäßen Verfahren dadurch, daß die Oxidschicht mittels eines Hochvakuum-Plasma-Beschichtungsverfahrens auf dem Substrat aufgebracht wird, wobei als Oxide die Oxide der Refraktärmetalle dienen.

Der Vorteil des erfindungsgemäßen Verfahrens liegt im wesentlichen darin, daß aufgrund des hohen Plasma-Metallanteils und der hohen Ionenenergien der verdampfenden Metalle, die Sauerstoff gebunden oder ungebunden enthalten, festhaftende und dichte Oberflächen erzeugt werden. Die Oberflächen sind zudem porenfrei, so daß es nicht zu den nachteiligen Korrosionswirkungen auf den Skelettimplantaten bekannter Art kommt. Das Calciumhydroxylapatit, das die zweite äußere Schicht auf dem Skelettimplantat bildet, ist auch Teil des Skelettes und des Zahnhartgewebes von Mensch und Säugetieren. Calciumhydroxylapatit hat somit die Eigenschaften, daß darin das Knochengewebe sehr gut einwachsen kann. Da das Calciumhydroxylapatit selbst in beschichteter Form mikroporös ist, wächst das Knochengewebe auch auf der darunterliegenden Oxidschicht an, d. h. durch die Calciumhydroxylapatitschicht hindurch. Es entstehen somit erfindungsgemäß osteointegrierende Oberflächen auf dem Substrat.

Als Hochvakuum-Plasma-Beschichtungsverfahren zur Aufbringung der Oxidschicht wird vorteilhafterweise das Lichtbogenverdampfungsverfahren verwendet. Bei diesem Verfahren handelt es sich um ein solches mit einem hohen Plasma-Metallanteil. Gleichermaßen vorteilhaft sind als Hochvakuum-Plasma-Beschichtungsverfahren das Laserverdampfungsverfahren oder das sogenannte Wire-Explosion-Verfahren, die sich ebenfalls durch einen hohen Plasma-Metallanteil auszeichnen.

Als Refraktärmetalle werden dabei vorzugsweise Titan, Zirkonium, Hafnium, Niob, Tantal, Wolfram oder Molybdän verwendet. Gemäß einer weiteren vorteilhaften Ausgestaltung des Verfahrens sind die Oxidschichten aus multinären Refraktärmetalloxydsystemen aufgebaut, wobei beispielsweise multinäre Systeme wie Titan, Zirkoniumoxyd die Oxidschicht bilden. Vorteilhafterweise kann aber auch für das multinäre System Titan-Nioboxyd benutzt werden.

Gemäß einer weiteren vorteilhaften Ausgestaltung des Verfahrens wird die Calciumhydroxylapatitschicht durch Aufspritzen auf die Oxidschicht aufgebracht.

Es kann auch vorteilhaft sein, die Calciumhydroxylapatitschicht durch Aufbrennen auf die Oxidschicht aufzubringen.

Unabhängig von der Art des Aufbringens der Calciumhydroxylapatitschicht auf der darunterliegenden Oxidschicht wird die Oxidschicht in bezug auf ihren Aufbau so gewählt, daß eine chemische Haftung zum Apatit entsteht und daß ein gutes Anwachsverhalten der biologischen Struktur, in die das Skelettimplantat eingesetzt wird, gewährleistet ist. Zudem ist das Oxid sehr gut mit dem biologischen Milieu verträglich.

Das Skelettimplantat aus Metall oder einer Metallegierung mit einer osteointegrierenden Oberfläche ist dadurch gekennzeichnet, daß die Oxidschicht eine unter Anwendung eines Hochvakuum-Plasma-Beschichtungsverfahrens aufgebrachte Schicht aus den Oxiden der Refraktärmetalle ist.

Der Vorteil eines derartigen Skelettimplantats besteht darin, daß einerseits eine besonders gute Integrationseigenschaft im biologischen Milieu gegeben ist und andererseits eine osteointegrierende Oberfläche auf dem Skelettimplantat geschaffen wird, die einen mechanisch nicht lösbaren, fest haftenden Verbund zwischen Knochen und Implantatoberfläche bildet. Vorteilhafterweise bilden die Oxide der Refraktärmetalle Titan, Zirkonium, Hafnium, Niob, Tantal, Wolfram und Molybdän die Oxide der ersten Schicht.

Die Erfindung wird nun unter Bezugnahme auf die nachfolgenden schematischen Zeichnungen anhand eines Ausführungsbeispieles beschrieben. Darin zeigen:

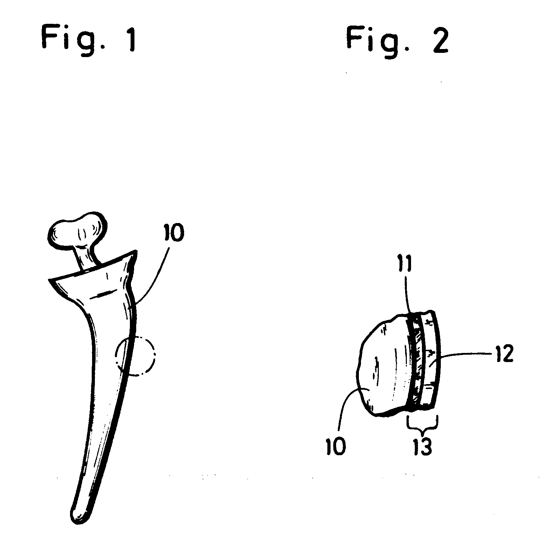

- Fig. 1: in der Seitenansicht ein Skelettimplantat zum Einsatz in einen Oberschenkelknochen des Menschen und
- Fig. 2: einen Ausschnitt aus der Oberfläche des in Fig. 1 dargestellten Skelettimplantats.

Das Skelettimplantat 10 kann beispielsweise auf der Basis von Titan bzw. Titanlegierungen, Kobalt-Chrom-Molybdänlegierungen oder auch aus beliebigen anderen Metallen oder Metallverbindungen oder Legierungen bestehen. Bei der Ausführung des Verfahrens wird zunächst eine erste Schicht 11 aus einem Oxid der Refraktärmetalle gebildet, was mittels eines Hochvakuum-Plasma-Beschichtungsverfahren erfolgt. Auf diese erste Schicht 11 wird eine zweite Schicht 12 aufgebracht, die aus Calciumhydroxylapatid CA₅[(OH) (PO₄)₃] besteht. Apatit in Form dieses Calciumhydroxylapatits ist ebenfalls natürlicher Bestandteil des Skelettes (Knochen, Zahnhartgewebe) von Mensch und Säugetieren.

Die erste Schicht 11 aus dem Oxid eines Refraktärmetalles und die zweite Schicht 12 aus Calciumhydroxylapatit bilden gemeinsam die äußere Beschichtung 13 des Skelettimplantats 10. Die erste Schicht 11 kann aber auch aus einer multinären Oxidschicht aufgebaut werden. Diese Oxidschichten aus multinären Metalloxydsystemen können beispielsweise Titan-Zirkoniumoxyd bzw. Titan-Nioboxyd sein. D. h. mit anderen Worten, daß die erste Schicht 11 selbst wiederum aus verschiedenen geeigneten mehrlagigen Schichten bzw. Schichtsystemen bestehen kann.

Das die zweite, äußere Schicht 12 bildende Calciumhydroxylapatit ist mikroporös, so daß das Knochengewebe, in das ein derart ausgebildetes Skelettimplantat 10 eingesetzt wird, sehr gut einwachsen kann. Das Knochengewebe wächst zudem auch auf der darunterliegenden ersten Oxidschicht 11 an, und zwar aufgrund der mikroporösen zweiten Schicht 12.

Ein erheblicher Vorteil des erfindungsgemäßen Verfahrens und des Skelettimplantats 10, das gemäß dem Verfahren hergestellt sein kann, besteht darin, daß bei sogenannten kapselnden Metallen, die das Skelettimplantat bilden, beispielsweise CoCrMo-Guß, die Oberfläche chemisch porendicht gegen das Gewebe abgedichtet ist, so daß eine hohe biologische Verträglichkeit erfindungsgemäß erreicht wird.

### Bezugszeichenliste

- 10: Skelettimplantat
- 11: erste Schicht
- 12: zweite Schicht
- 13: Beschichtung

## Patentansprüche

1. Verfahren zur Herstellung von osteointegrierenden Oberflächen auf Skelettimplantaten aus Metall oder Metallegierungen, wobei zuerst eine Oxidschicht auf dem Skelettimplantat aufgebracht wird und nachfolgend darauf eine Calciumhydroxylapatitschicht, dadurch gekennzeichnet, daß die Oxidschicht mittels eines Hochvakuum-Plasma-Beschichtungsverfahrens aufgebracht wird, wobei als Oxide die Oxide der Refraktärmetalle dienen.

2. Verfahren nach Anspruch 1, dadurch gekennzeichnet, daß die Refraktärmetalle Titan, Zirkonium, Hafnium, Niob, Tantal, Wolfram oder Molybdän sind.

3. Verfahren nach einem oder beiden der Ansprüche 1 oder 2, dadurch gekennzeichnet, daß die Calciumhydroxylapatitschicht durch Aufspritzen auf die Oxidschicht aufgebracht wird.

4. Verfahren nach einem oder mehreren der Ansprüche 1 bis 3, dadurch gekennzeichnet, daß die Calciumhydroxylapatitschicht durch Aufbrennen auf die Oxidschicht aufgebracht wird.

5. Skelettimplantat aus Metall oder einer Metallegierung mit einer osteointegrierenden Oberfläche, wobei auf dem Skelettimplantat (10) eine aus wenigstens zwei Schichten (11, 12) bestehende Beschichtung (13) ausgebildet ist, und wobei die unmittelbar auf dem Skeletttimplantat (10) ausgebildete erste Schicht (11) eine Oxidschicht ist und die außen ausgebildete zweite Schicht (12) aus Calciumhydroxylapatit besteht, dadurch gekennzeichnet, daß die Oxidschicht eine unter Anwendung eines Hochvakuum-Plasma-Beschichtungsverfahrens aufgebrachte Schicht (11) aus den Oxiden der Refraktärmetalle ist.

6. Skelettimplantat nach Anspruch 5, dadurch gekennzeichnnet, daß die Refraktärmetalle Titan, Zirkonium, Hafnium, Niob, Tantal, Wolfram oder Molybdän sind.

## Claims

1. Process for producing osteo-integrating surfaces on skeletal implants made of metal or metal alloys, wherein an oxide layer is initially applied to the skeletal implant and a calcium hydroxyl apatite layer is subsequently applied on top of it, characterised in that the oxide layer is applied by means of a high-vacuum plasma coating process, with the oxides of the refractory metals serving as oxides.

2. Process according to Claim 1, characterised in that the refractory metals are titanium, zirconium, hafnium, niobium, tantalum, tungsten or molybdenum.

3. Process according to one or both of Claims 1 or 2, characterised in that the calcium hydroxyl apatite layer is applied to the oxide layer by spray application.

4. Process according to one or more of Claims 1 to 3, characterised in that the calcium hydroxyl apatite layer is applied to the oxide layer by baking it onto the oxide layer.

5. Skeletal implant made of metal or a metal alloy with an osteo-integrating surface, wherein a coating (13) comprising at least two layers (11,12) is formed on the skeletal implant (10) and wherein the first layer (11) formed immediately on the skeletal implant (10) is an oxide layer and the second layer (12) formed on the outside comprises calcium hydroxyl apatite, characterised in that the oxide layer is a layer (11) of the oxides of the refractory metals applied using a high-vacuum plasma coating process.

6. Skeletal implant according to Claim 5, characterised in that the refractory metals are titanium, zirconium, hafnium, niobium, tantalum, tungsten or molybdenum.

## Revendications

1. Procédé de préparation de surfaces d'intégration osseuse sur des implants de squelette métalliques ou en alliages métalliques, en applicant d'abord une couche d'oxyde sur l'implant de squelette, puis par-dessus, une couche d'hydroxyapatite de calcium, caractérisé en ce qu'on applique la couche d'oxyde à l'aide d'un procédé de revêtement au plasma sous vide poussé, les oxydes de métaux réfractaires servant d'oxydes.

2. Procédé selon la revendication 1, caractérisé en ce que les métaux réfractaires sont le titane, le zirconium, l'hafnium, le niobium, le tantale, le tungstène ou le molybdène.

3. Procédé selon une ou plusieurs des revendication 1 ou 2, caractérisé en ce que l'on applique la couche d'hydroxyapatite de calcium sur la couche d'oxyde au pistolet.

4. Procédé selon une ou plusieurs des revendications 1 à 3, caractérisé en ce que l'on applique la couche d'hydroxyapatite de calcium sur la couche d'oxyde par cuisson.

5. Implants de squelette métalliques ou en un alliages métalliques avec une surface d'intégration osseuse, un revêtement (13) constitué d'au moins deux couches (11, 12) étant formé sur l'implant de squelette (10), et la première couche formé directement sur l'implantat de squelette (10) étant une couche d'oxyde et la deuxième couche externe (12) formée par dessus étant en hydroxyapatite de calcium, caractérisé en ce que la couche d'oxyde étant une couche (11) d'oxydes de métaux réfractaires appliquée à l'aide d'un procédé de revêtement au plasma sous vide poussé.

6. Implant de squelette selon la revendication 5, caractérisé en ce que les métaux réfractaires sont le titane, le zirconium, l'hafnium, le niobium, le tantale, le tungstène ou le molybdène.
